# EUROPÄISCHE PATENTSCHRIFT

(11) **EP 3 292 885 B1**
(45) Veröffentlichungstag und Bekanntmachung des Hinweises auf die Patenterteilung: **17.07.2019**
(21) Anmeldenummer: 17183909.5
(22) Anmeldetag: 31.07.2017
(51) Int. Cl.: A61N 1/05, A61B 5/042, H05K 1/02

(54) **DEHNBAHRE ELEKTRODENLEITERANORDNUNG UND MEDIZINISCHES IMPLANTAT**
STRETCHABLE ELECTRODE CONDUCTOR ASSEMBLY AND MEDICAL IMPLANT
SYSTÈME DE CONDUCTEUR D'ÉLECTRODE ÉLASTIQUE ET IMPLANT MÉDICAL

(30) Priorität: 06.09.2016 US 201662383604 P
(43) Veröffentlichungstag der Anmeldung: 14.03.2018
(73) Patentinhaber: BIOTRONIK SE & Co. KG, 12359 Berlin (DE)
(72) Erfinder: Bihler, Eckardt, 8406 Winterthur (CH); Hauer, Marc, 8610 Uster (CH); Schulze, Daniel, 8303 Bassersdorf (CH)
(74) Vertreter: Keck, Hans-Georg

(56) Entgegenhaltungen:
- EP-A1- 2 246 092
- DE-A1-102004 035 903
- US-A1- 2013 312 258
- US-A1- 2015 065 840
- US-A1- 2015 157 852
- US-A1- 2016 165 719

## Beschreibung

Die Erfindung betrifft eine dehnbare Elektrodenleiteranordnung für ein medizinisches Implantat, die mindestens einen zickzack- oder mäanderförmigen Leiterzug auf einem isolierenden Träger und mit einer isolierenden, mit dem Träger unter Einbettung des Leiterzuges dicht verbundenen Abdeckung aufweist. Sie betrifft des Weiteren ein medizinisches Implantat mit einer solchen Elektrodenleiteranordnung.

Medizinelektronische Implantate, wie etwa Herzschrittmacher, implantierte Kardioverter, Cochlearimplantate oder Neurostimulatoren, üben eine Wirkung auf reizbares Körpergewebe des Patienten über Elektroden (auch Elektrodenpole genannt) aus, die in Kontakt mit dem Körpergewebe sind. Umgekehrt können über mit geeignetem Körpergewebe in Kontakt stehende Elektroden körpereigene Potentiale erfasst und für eine diagnostische Auswertung verfügbar gemacht werden.

Die Elektroden bzw. Elektrodenpole sind in den meisten Fällen an langgestreckten Elektrodenleitungen oder speziellen Kathetern angebracht, können aber bei bestimmten Anordnungen auch am Stimulationsgerät selbst angebracht oder in Form von netz- oder mattenförmigen flächigen Anordnungen verlegt sein. Bekannt sind hierbei auch elastisch dehnbare Elektrodenanordnungen, die für bestimmte Anwendungen vorteilhaft sind. Ein expandierbares Implantat mit integrierter Sensorik, welches eine solche dehnbare Elektrodenanordnung aufweist, wird in der WO 02/058549 A1 beschrieben.

Bei den bisher bekannten dehnbaren Elektrodenarrays werden die elektrisch leitenden Schichten auf dehnbaren Materialien aufgebracht und mit dehnbaren Materialien verkapselt. Die Leiterbahnen werden dabei in Mäandern auf dem dehnbaren Basismaterial geführt. Bei der Dehnung entstehen Zugkräfte zwischen den Metallschichten und dem dehnbaren Basismaterial, die die maximale Anzahl der Dehnzyklen und die maximalen Dehnungen limitieren. Durch die Zugkräfte können sich die Leiterzüge vom Basismaterial abheben. Es können Migrationspfade für Elektrolyte an Hohlräume längs den Leiterzügen entstehen, die zum Ausfall bzw. einer Reduktion der Lebensdauer der Strukturen führen können.

Dokument US 2015/65840 A1 beschreibt dehnbare gedruckte Leiterplatten die in Serpentinen ausgebildete elektrische Leiter umfassen, die in dehnbares Material eingebettet sind. Dieses Dehnbare Material selbst weist eine Serpentinenartige Form auf. Diese dehnbare gedruckte Leiterplatte ist für tragbare elektrische oder elektronische Geräte einsetzbar.

Dokument US 2013/312258 A1 beschreibt eine dehnbare Elektrodenleitung aus flexiblem Material, das über entlang der Elektrodenleitung verlaufende elektrische Leiter enthält. Die Dehnbarkeit wird durch eine Vielzahl von Biegungen des Materials zu einer Wellenstruktur erreicht.

Dokument US 2015/157852 A1 beschreibt eine flexible Elektrodenleitung für ein Cochlea Implantat.

Dokument US 2016/165719 A1 beschreibt ein Patch mit einer faltbaren Leiterplatte.

Der Erfindung liegt daher die Aufgabe zugrunde, eine funktionssichere und für Langzeitbetrieb im implantierten Zustand geeignete dehnbare Elektrodenleiteranordnung anzugeben. Es soll weiterhin ein entsprechend zuverlässiges medizinisches Implantat bereitgestellt werden.

Diese Aufgabe wird durch eine Elektrodenleiteranordnung mit den Merkmalen des Anspruchs 1 bzw. ein medizinisches Implantat mit den Merkmalen des Anspruchs 12 gelöst.

Zweckmäßige Fortbildungen des Erfindungsgedankens sind Gegenstand der abhängigen Ansprüche.

Die Erfindung schließt den Gedanken ein, den Träger der Elektrodenanordnung aus einem im Wesentlichen nicht dehnbaren Material zu bilden, welches bei einer Dehnung der Anordnung auf die Grenzfläche zu den Leiterzügen nur noch geringe Zugkräfte ausübt. Damit kann eine höhere Lebensdauer auch bei einem Betrieb mit häufigen Dehnungen, auch um relativ große Beträge, gewährleistet werden. Die Erfindung schließt weiterhin den Gedanken ein, die erforderliche Dehnbarkeit der Anordnung als Ganzes dadurch zu realisieren, dass der Träger in Anpassung an die Kontur des Leiterzuges oder der Leiterzüge zickzack- oder mäanderförmig zugeschnitten ist. Die Dehnbarkeit des Trägers wird also, ebenso wie bei den Leiterzügen, durch die spezielle geometrische Konfiguration realisiert.

Vereinfacht ausgedrückt, kann man sich die erfindungsgemäße Anordnung als eine Art mehrlagiges zickzack- oder mäanderförmiges Flachbandkabel vorstellen, welches für den bestimmungsgemäßen Einsatz bei medizinischen Implantaten insbesondere mit biokompatiblen Materialien ausgeführt ist. Unter einer mäanderförmigen Anordnung sind verschiedene konkrete Konfigurationen des Trägers zu verstehen, bei denen die Kontur beispielsweise durch alternierend ausgeschnittene Rechtecke oder Trapeze oder durch U- oder auch Ω-förmige Ausschnitte definiert sein kann. Die Außenkontur des entsprechend geformten Trägers und der Abdeckung muss nicht streng zur Leiterzug-Konfiguration korrespondieren, sondern kann durchaus etwas von jener abweichen, solange die geometrie-bedingte Dehnbarkeit des Trägers (und optional der Abdeckung) sichergestellt ist.

Die Abdeckung weist auch ein im Wesentlichen nicht dehnbares Material auf und ist in Anpassung an die Kontur des Leiterzuges oder der Leiterzüge zickzack- oder mäanderförmig zugeschnitten. Dies wird aus derzeitiger Sicht die in der Praxis realisierte Ausführung sein, grundsätzlich kann für die Abdeckung aber auch ein stärker dehnbares Material als für den Träger der Anordnung benutzt werden.

Des Weiteren wird die vorgeschlagene Elektrodenleiteranordnung in der praktischen Ausführung normalerweise mindestens einen elektrisch mit dem oder mindestens einem Leiterzug verbundenen Elektrodenpol - typischerweise mehrere Elektrodenpole - aufweisen, der in den Träger oder die Abdeckung eingebettet ist und eine freie Oberfläche hat. Alternativ kann der Elektrodenpol oder können die Elektrodenpole auf der freien Oberfläche des Trägers oder der Abdeckung angeordnet und über eine Durchgangskontaktierung mit dem zugehörigen eingebetteten Leiterzug verbunden sein.

Grundsätzlich kann aber die vorgeschlagene Elektrodenanordnung auch ohne integrierte Elektrodenpole genutzt werden, insbesondere indem sie an separate Elektrodenpole angeschlossen wird, die etwa am distalen Ende einer Elektrodenleitung als Tip- oder Ringelektroden vorgesehen sein können.

In weiteren Ausführungen weist die vorgeschlagene Elektrodenleiteranordnung eine Mehrzahl parallel verlaufender Leiterzüge und an den Verlauf der Leiterzüge angepasste Ausschnitte im Träger und auch in der Abdeckung auf. Hierbei kann die Anordnung insbesondere eine netzartige Konfiguration von sich schneidenden Leiterzügen und im Wesentlichen polygonal, z. B. rechteckige oder parallelogrammförmige, Ausschnitte im Träger und optional auch in der Abdeckung aufweisen. Wichtig ist hierbei, dass die erwähnten Ausschnitte im Träger und gegebenenfalls auch der Abdeckung zickzack- bzw. mäanderförmige Ränder entsprechend dem Verlauf der angrenzenden Leiterzüge haben, damit die angestrebte Dehnbarkeit erreicht wird.

In einer weiteren Ausgestaltung weist eine Elektrodenleiteranordnung der zuletzt genannten Art ein matrixförmiges Array von Elektrodenpolen auf, die insbesondere an Kreuzungs- oder Verbindungspunkten mehrerer Leiterzüge angeordnet sind. Mit einer solchen Anordnung lässt sich vorteilhaft eine Stimulation und/oder sensorische Abtastung größerer Flächenabschnitte eines menschlichen oder tierischen Körpers realisieren.

Im Kontext der vorliegenden Erfindung ist es wesentlich, dass für den Träger ein gegenüber bisher verwendeten Materialien deutlich weniger dehnbares Material eingesetzt wird. Wenn dieses als ein im Wesentlichen nicht dehnbares Material qualifiziert wird, bedeutet dies allerdings nicht, dass es keinerlei Dehnbarkeit aufweisen darf. Geeignete Materialien für den Träger und/oder die Abdeckung sind ein flüssigkristallines Polymer, LCP, oder ein Polyimid, PI. Je nach Anwendungssituation können aber auch andere Typen/Klassen von Kunststoffen zum Einsatz kommen.

In aus derzeitiger Sicht sinnvollen Ausführungen liegt die Dicke des Trägers und/oder der Abdeckung im Bereich zwischen 5 und 100 µm und die Dicke des oder jedes Leiterzuges im Bereich zwischen 1 und 20 µm. Es versteht sich, dass die Nicht-Dehnbarkeit von Träger und Abdeckung umso eher gewährleistet ist, je dicker die Materialschichten sind. Eine größere Dicke bringt aber zwangsläufig eine höhere Biegesteifigkeit und andere Nachteile mit sich, so dass bei der Ausführung der Erfindung letztlich ein Kompromiss zwischen der materialspezifischen Dehnbarkeit und der Materialdicke im Hinblick auf die Erzielung der oben erwähnten Vorteile und zur Vermeidung von Anwendungs-Nachteilen gefunden werden muss.

Weiterhin ist bei der praktischen Ausführung aus derzeitiger Sicht vorgesehen, dass die Breite des oder jedes Leiterzuges im Bereich zwischen 2 und 500 µm, spezieller zwischen 10 und 100, µm liegt und die lokale Breite des Trägers größer als die Breite des Leiterzuges ist und im Bereich zwischen 25 und 250 µm liegt. Für spezielle Anwendungen sind aber grundsätzlich auch andere Breiten denkbar, sofern durch entsprechende Wahl der Materialart und -dicke eine erfindungsgemäße Funktion sichergestellt ist.

Der Verbund aus Träger, Leiterzug bzw. Leiterzügen und Abdeckung ist auf eine elastisch verformbare, insbesondere dehnbare flächige Basis aufgebracht oder in eine derartige Basis eingebettet. Hierunter wird im Normalfall eine zusätzliche Basis (also vierte Materialschicht) verstanden, grundsätzlich ist aber auch eine Funktions-Kombination aus (in diesem Fall dehnbarer) Abdeckung und Basis in einer einzelnen Komponente denkbar. Die Basis kann ein Polyurethan- oder Silikonmaterial oder einen Kautschuk aufweisen.

In Ausgestaltungen dieser Ausführung ist die Basis zylindrisch oder ballonartig räumlich geformt, oder sie weist einen zylindrischen oder ballonförmigen Abschnitt auf. Mit derartigen Ausgestaltungen lassen sich beispielsweise neuartige Elektrodenleitungen oder Ballonkatheteranordnungen mit Stimulations- bzw. Sensing-Funktion realisieren.

In einer Ausgestaltung des vorgeschlagenen Implantats umfasst dieses eine in die Elektrodenleiteranordnung eingefügte zusätzliche Funktionskomponente, etwa eine elektronische Steuerungs- und/oder Auswertungskomponente. Diese kann insbesondere zwischen der eigentlichen Elektrodenleiteranordnung und einer diese tragenden oder einhüllenden dehnbaren Basis platziert sein.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A und 1B: eine schematische Schnittansicht bzw. Draufsicht einer Elektrodenleiteranordnung gemäß einer Ausführung der Erfindung,
- Fig. 2: eine schematische Draufsicht einer Elektrodenleiteranordnung gemäß einer weiteren Ausführungsform der Erfindung,
- Fig. 3: eine schematische Draufsicht einer Elektrodenleiteranordnung gemäß einer weiteren Ausführungsform der Erfindung,
- Fig. 4: eine schematische Darstellung eines speziellen Einsatzes der Elektrodenleiteranordnung nach Fig. 3,
- Fig. 5: eine schematische Darstellung eines medizinischen Implantats gemäß einer Ausführungsform der Erfindung,
- Fig. 6A und 6B: eine perspektivische Darstellung sowie eine Teilansicht eines medizinischen Implantats gemäß einer weiteren Ausführung der Erfindung und
- Fig. 7A und 7B: schematische Schnittansichten eines medizinischen Implantats gemäß einer weiteren Ausführungsform der Erfindung.

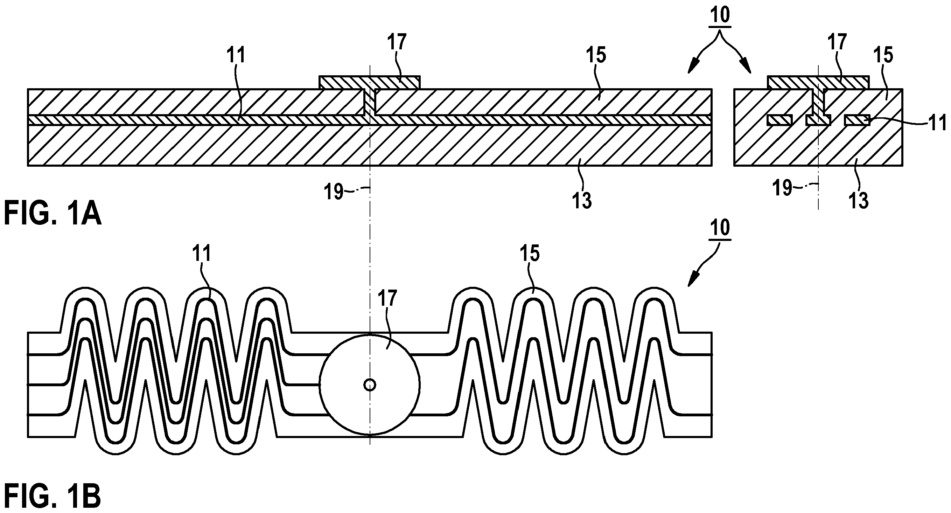

Fig. 1A und 1B zeigen eine dreischichtige Elektrodenleiteranordnung 10, bei der mehrere parallel zickzackförmig verlaufende Leiterzüge 11 zwischen eine erste nicht dehnbare Polymerschicht (Träger) 13 und eine zweite nicht dehnbare Polymerschicht (Abdeckung) 15 eingebettet sind, wobei die erste und zweite Polymerschicht 13, 15 eine Außenkontur haben, die in Anpassung an den zickzackförmigen Verlauf der Leiterzüge 11 zugeschnitten ist. Das Zuschneiden kann etwa mittels Laser oder auch durch Fräsen oder Plasmaätzen oder gegebenenfalls mittels eines Stanzwerkzeugs erfolgen.

Bei der dargestellten Ausführung sind in einem Mittenbereich zwischen beidseitigen Leiterzügen und zugeordneten Träger/Abdeckungs-Bereichen ein auf die Abdeckung 15 aufgelegter scheibenförmiger Elektrodenpol 17 und eine Durchgangskontaktierung (Via) 19 vorgesehen, die den Elektrodenpol mit den Leiterzügen verbindet.

Durch die zickzackförmige Anordnung der Leiterzüge und den hierzu korrespondierenden zickzackförmigen Zuschnitt des Trägers 13 und der Abdeckung 15 lassen sich die beidseits des Mittelteils mit dem Elektrodenpol 17 angrenzenden Bereiche der Elektrodenleiteranordnung 10 problemlos in Längsrichtung elastisch dehnen, ohne dass wiederholte und auch ausgebprägte Dehnungen zu den weiter oben beschriebenen Nachteilen führen, wie sie bei Träger/Abdeckungs-Verbunden aus intrinsisch dehnbaren Materialien nach längerer Zeit auftreten.

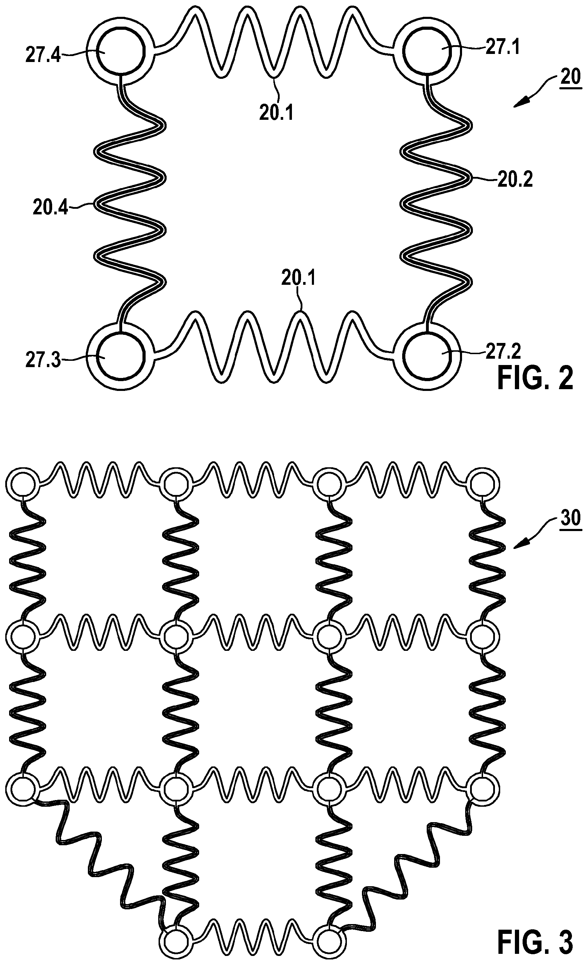

Fig. 2 zeigt in Draufsicht eine weitere beispielhafte Elektrodenleiteranordnung 20, welche in der Grundform eines Quadrates vier Träger/Leiterzug/Abdeckungs-Stränge 20.1 - 20.4 und an den Eckpunkten jeweils einen Elektrodenpol 27.1 - 27.4 umfasst.

Fig. 3 zeigt eine weitere beispielhafte Elektrodenleiteranordnung 30, die in einer 2 x 3 Matrixanordnung sechs Teilanordnungen der in Fig. 2 gezeigten Art und zusätzlich eine rechteckförmige und beidseits dieser zwei dreieckförmige Teilanordnungen umfasst.

Um die Struktur mechanisch zusätzlich zu stabilisieren und besser an das Gewebe anzupassen, kann die ausgeschnittene Mäanderstruktur in ein dehnbares Polymer eingebettet werden. Dieses Polymer kann entweder als geschlossener Film vorliegen oder durchbrochen sein, damit die Struktur ins Körpergewebe einwachsen kann. Die Einbettung kann entweder durch Einpressen bei erhöhter Temperatur und Druck erfolgen, wobei die Temperatur über den Glaspunkt des zweiten Polymer (z. B. PU), aber noch unterhalb des Glaspunktes des ersten Polymers (z. B. LCP) angehoben wird, oder anderseits durch Abformen mit einem zweiten Polymer (z. B. Silikonharz) in einer Form erfolgen.

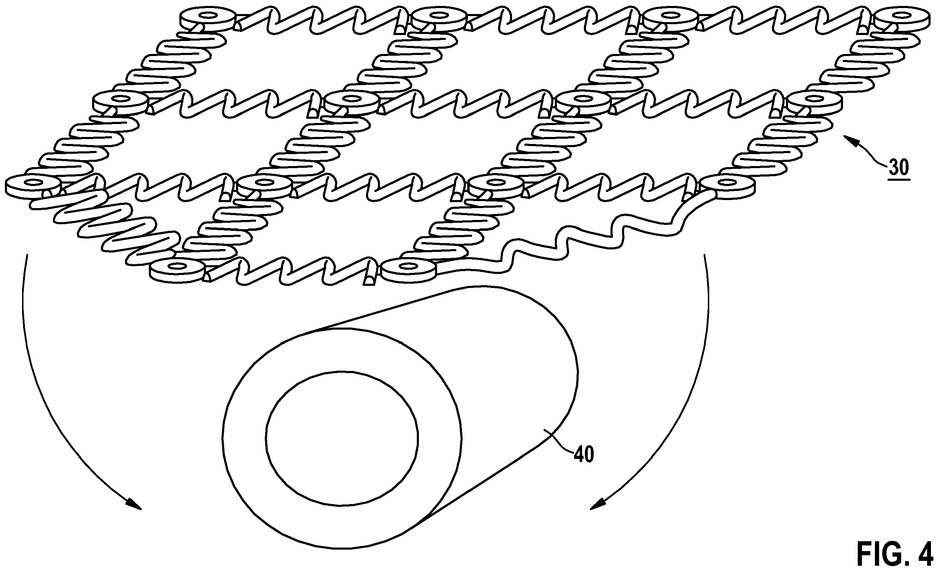

Fig. 4 zeigt schematisch, wie die in Fig. 3 in Draufsicht gezeigte Anordnung 30 auf den Umfang einer schlauchförmigen Basis 40 aufgebracht wird. Auf diese Weise lässt sich beispielsweise ein Katheter mit einem regulären Array von Elektrodenpolen auf seinem Umfang ausrüsten, um beispielsweise ein Gefäß in einem längeren Abschnitt zu stimulieren oder dort Gewebspotentiale abzufühlen. Das Aufbringen der Elektrodenleiteranordnung 30 kann durch Aufpressen bei erhöhter Temperatur erfolgen, oder es kann bei Nutzung eines Silikonschlauchs als Basis 40 auch durch Einlegen der Elektrodenleiteranordnung in eine entsprechende Form und Ausfüllen derselben mit dem Silikonmaterial erfolgen.

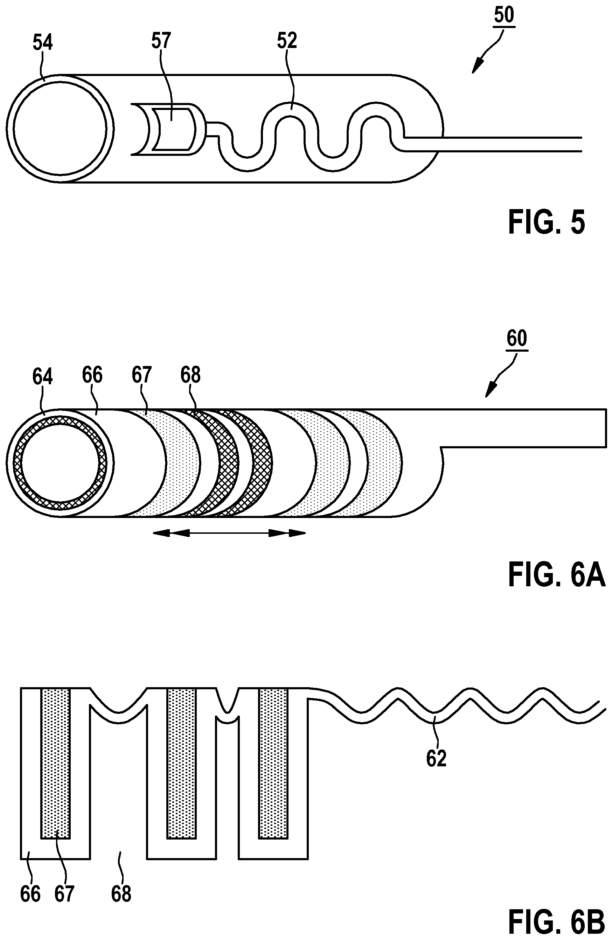

Fig. 5 zeigt schematisch, als weiteres Beispiel eines Einsatzes der erfindungsgemäßen Elektrodenleiteranordnung, einen distalen Endabschnitt einer Elektrodenleitung 50, der mit einem im wesentlichen rechteckförmigen Elektrodenpol 57 bestückt ist. Eine mäanderförmige Zuleitung 52, die durch einen Träger/Leiterzug/Abdeckung-Verbund der weiter oben beschriebenen Art realisiert ist, ist auf einen dehnbaren Silikon- oder PU-Schlauch 54 als Grundkörper aufgebracht.

Eine modifizierte Ausführung eines elektrisch wirksamen Katheters 60 ist in Fig. 6A und 6B in einer schematischen perspektivischen Darstellung bzw. Detailansicht gezeigt. Grundkörper ist hier wiederum ein dehnbarer PU- oder Silikonschlauch 64, auf den drei Ringelektroden 67 jeweils auf einem Substrat 66 so aufgebracht sind, dass dazwischen Dehnungsfugen 68 verbleiben. Angeschlossen sind die Ringelektroden über eine wiederum zickzack- bzw. mäanderförmig verlaufende Zuleitungsstruktur 62 der erfindungsgemäßen Art.

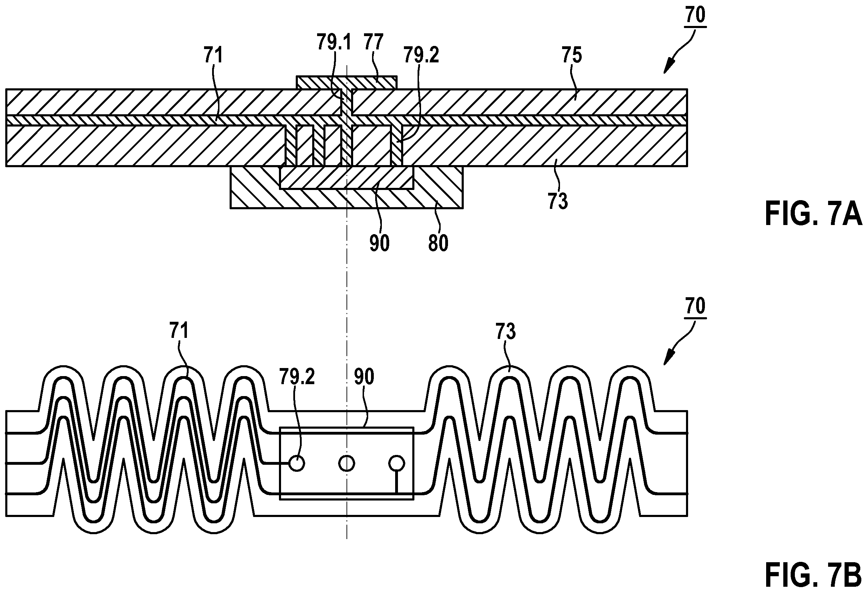

Fig. 7A und 7B zeigen in schematischen Schnittansichten in zueinander senkrechten Schnittebenen ein weiteres medizinisches Implantat 70, welches grundsätzlich sehr ähnlich aufgebaut ist wie die in Fig. 1A und 1B gezeigte und weiter oben beschriebene Elektrodenleiteranordnung 10. Die Bezugsziffern für die einzelnen Teile sind daher an diejenigen in Fig. 1A/1B angelehnt. Die diesbezügliche Beschreibung der Teile wird hier nicht wiederholt.

Zusätzlich umfasst die Anordnung eine dritte Polymerschicht 80, die unterhalb eines Abschnitts des Trägers 73 angeordnet ist, und in diese ist eine Funktionskomponente 90 eingebettet. Die Komponente 90 ist über zweite Durchgangskontaktierungen 79.2 an die Leiterzüge 71 der Anordnung angeschlossen, die sich von den Leiterzügen in die entgegengesetzte Richtung, relativ zu ersten Durchgangskontaktierungen 79.1 des Elektrodenpols 77, erstrecken. Bei der zusätzlichen Komponente kann es sich beispielsweise um Sensorik oder Ultraschallwandler handeln, die zur Signalbeaufschlagung- oder Ableitung über die Leiterzüge der Elektrodenleiteranordnung anzuschließen sind.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Dehnbare Elektrodenleiteranordnung (10; 20; 30; 52; 62) für ein medizinisches Implantat (50; 60; 70), die mindestens einen zickzack- oder mäanderförmigen Leiterzug (11; 71) auf einem isolierenden Träger (13; 73) und mit einer isolierenden, mit dem Träger unter Einbettung des Leiterzuges dicht verbundenen Abdeckung (15; 75) aufweist,
wobei der Träger ein im Wesentlichen nicht dehnbares Material aufweist,
wobei auch die Abdeckung (15; 75) ein im Wesentlichen nicht dehnbares Material aufweist,
wobei der Verbund (30; 52; 62) aus Träger, Leiterzug bzw. Leiterzügen und Abdeckung auf eine elastisch verformbare, insbesondere dehnbare flächige Basis (40; 50; 60) aufgebracht oder in eine derartige Basis eingebettet ist,
**dadurch gekennzeichnet, dass** der Träger und auch die Abdeckung in Anpassung an die Kontur des Leiterzuges oder der Leiterzüge zickzack- oder mäanderförmig zugeschnitten sind und dadurch als Ganzes dehnbarsind.

2. Elektrodenleiteranordnung nach Anspruch 1, welche mindestens einen elektrisch mit dem oder mindestens einem Leiterzug verbundenen Elektrodenpol (17; 27.1 - 27.4; 57; 67; 77) aufweist, der in den Träger (13; 73) oder die Abdeckung (15; 75) eingebettet ist und eine freie Oberfläche hat.

3. Elektrodenleiteranordnung nach Anspruch 1, welche mindestens einen elektrisch mit dem oder mindestens einem Leiterzug verbundenen Elektrodenpol (17; 27.1 - 27.4; 57; 67; 77) aufweist, der auf der freien Oberfläche des Trägers (13; 73) oder der Abdeckung (15; 75) angeordnet und über eine Durchgangskontaktierung mit dem eingebetteten Leiterzug verbunden ist.

4. Elektrodenleiteranordnung nach einem der vorangehenden Ansprüche, welche eine Mehrzahl parallel verlaufender Leiterzüge (11; 71) und an den Verlauf der Leiterzüge angepasste Ausschnitte im Träger (13; 73) und auch in der Abdeckung (15; 75) aufweist.

5. Elektrodenleiteranordnung nach Anspruch 4, welche eine netzartige Anordnung (50) von sich schneidenden Leiterzügen und im Wesentlichen polygone Ausschnitte im Träger und optional auch in der Abdeckung aufweist.

6. Elektrodenleiteranordnung nach Anspruch 5, mit einem matrixförmigen Array von Elektrodenpolen, die insbesondere an Kreuzungs- oder Verbindungspunkten mehrerer Leiterzüge angeordnet sind.

7. Elektrodenleiteranordnung nach einem der vorangehenden Ansprüche, wobei der Träger (13; 73) und/oder die Abdeckung (15; 75) ein flüssigkristallines Polymer, LCP, oder ein Polyimid, PI, aufweist.

8. Elektrodenleiteranordnung nach einem der vorangehenden Ansprüche, wobei die Dicke des Trägers (13; 73) und/oder der Abdeckung (15; 75) im Bereich zwischen 5 und 100 µm und die Dicke des oder jedes Leiterzuges (11; 71) im Bereich zwischen 0,1 und 20 µm liegt.

9. Elektrodenleiteranordnung nach einem der vorangehenden Ansprüche, wobei die Breite des oder jedes Leiterzuges (11; 71) im Bereich zwischen 2 und 500 µm liegt und die lokale Breite des Trägers (13; 73) größer als die Breite des Leiterzuges ist und im Bereich zwischen 25 und 250 µm liegt.

10. Elektrodenleiteranordnung nach Anspruch 1, wobei die Basis (40; 50; 60) ein Polyurethan-, Silikonmaterial oder Kautschuk aufweist.

11. Elektrodenleiteranordnung nach Anspruch 1 oder Anspruch 10, wobei die Basis (40; 50; 60) zylindrisch oder ballonartig räumlich geformt ist oder einen zylindrischen oder ballonförmigen Abschnitt aufweist.

12. Medizinisches Implantat (50; 60; 70), insbesondere Elektrodenleitung, Elektrodenmatte oder Ballonkatheter, mit einer Elektrodenleiteranordnung (10; 20; 30; 52; 62) nach einem der vorangehenden Ansprüche.

13. Medizinisches Implantat (70) nach Anspruch 12, wobei der Elektrodenleiteranordnung eine zusätzliche Funktionskomponente (90), insbesondere eine elektronische Steuerungs- und/oder Auswertungskomponente, zugeordnet und diese insbesondere zwischen der Elektrodenleiteranordnung und einer zugeordneten Basis (80) platziert ist.

## Claims

1. A stretchable electrode conductor arrangement (10; 20; 30; 52; 62) for a medical implant (50; 60; 70), this stretchable electrode conductor arrangement having at least one zigzag or meandering conductor track (11; 71) on an insulating support (13; 73) and an insulating cover (15; 75) that is tightly connected with the support, embedding the conductor track,
the support comprises an essentially non-stretchable material,
the cover (15; 75) also comprises an essentially non-stretchable material,
the composite (30; 52; 62) consisting of support, conductor track(s) and cover being put on an elastically deformable, especially stretchable planar base (40; 50; 60) or being embedded in such a base,
**characterized in that** the support and also the cover are cut to size in a zigzag or meandering shape to adapt them to the contour of the conductor track(s), making them stretchable as a whole.

2. An electrode conductor arrangement according to claim 1, which comprises at least one electrode pole (17; 27.1 - 27.4; 57; 67; 77) which is electrically connected with the at least one conductor track and which is embedded in the support (13; 73) or the cover (15; 75) and has an exposed surface.

3. An electrode conductor arrangement according to claim 1, which comprises at least one electrode pole (17; 27.1 - 27.4; 57; 67; 77) which is electrically connected with the or at least one conductor track and which is arranged on the exposed surface of the support (13; 73) or the cover (15; 75) and is connected with the embedded conductor track through a via contact.

4. The electrode conductor arrangement according to any one of the preceding claims, which has multiple parallel conductor tracks (11; 71) and cutouts in the support (13; 73), and also in the cover (15; 75), that are adapted to the course of the conductor tracks.

5. The electrode conductor arrangement according to claim 4, which has a netlike arrangement (50) of intersecting conductor tracks and essentially polygonal cutouts in the support and optionally also in the cover.

6. The electrode conductor arrangement according to claim 5, with a matrix-shaped array of electrode poles, in particular electrode poles that are arranged at crossing points or connection points of multiple conductor tracks.

7. The electrode conductor arrangement according to any one of the preceding claims, wherein the support (13; 73) and/or the cover (15; 75) comprises a liquid crystal polymer (LCP), or a polyimide (PI).

8. the electrode conductor arrangement according to any one of the preceding claims, wherein the thickness of the support (13; 73) and/or the cover (15; 75) is in the range between 5 and 100 µm and the thickness of the or every conductor track (11; 71) is in the range between 1 and 20 µm.

9. The electrode conductor arrangement according to any one of the preceding claims, wherein the width of the, or every, conductor track (11; 71) is in the range between 2 and 500 µm, and the local width of the support (13; 73) is greater than the width of the conductor track and is in the range between 25 and 250 µm.

10. The electrode conductor arrangement according to claim 1, wherein the base (40; 50; 60) comprises a polyurethane or silicone material or a rubber.

11. The electrode conductor arrangement according to claim 1 or claim 10, wherein the base (40; 50; 60) is spatially shaped as a cylinder or balloon, or it has a cylindrical or balloon-shaped section.

12. A medical implant (50; 60; 70), especially an electrode lead, electrode mat, or balloon catheter, with an electrode conductor arrangement (10; 20; 30; 52; 62) according to any one of the preceding claims.

13. the medical implant (70) according to claim 12, wherein the electrode conductor arrangement is associated with an additional functional component (90), especially an electronic control and/or evaluation component, this component being placed especially between the electrode conductor arrangement and an associated base (80).

## Revendications

1. Ensemble de conducteurs d'électrode (10 ; 20 ; 30 ; 52 ; 62) étirable pour un implant (50 ; 60 ; 70) médical qui présente au moins une piste conductrice (11 ; 71) en forme de zigzag ou de méandres sur un support (13 ; 73) isolant et avec une enveloppe (15 ; 75) isolante reliée de manière étanche avec le support moyennant une incorporation de la piste conductrice,
où le support présente essentiellement un matériau non étirable,
où l'enveloppe (15 ; 75) présente également un matériau essentiellement non étirable,
où le composite (30 ; 52 ; 62) à base du support, de la piste conductrice, respectivement des pistes conductrices et l'enveloppe sont rapportés sur une base (40 ; 50 ; 60) plane déformable élastiquement, notamment étirable, ou sont incorporés dans une telle base,
**caractérisé en ce que** le support et également l'enveloppe sont découpés en forme de zigzags ou de méandres en s'ajustant au contour de la piste conductrice ou des pistes conductrices et sont ainsi étirables dans leur ensemble.

2. Ensemble de conducteurs d'électrode selon la revendication 1, lequel présente au moins un pôle d'électrode (17 ; 27.1 à 27.4 ; 57 ; 67 ; 77) relié électriquement avec la ou l'au moins une piste conductrice, qui est incorporé dans le support (13 ; 73) ou dans l'enveloppe (15 ; 75) et a une surface libre.

3. Ensemble de conducteurs d'électrode selon la revendication 1, lequel présente au moins un pôle d'électrode (17 ; 27.1 à 27.4 ; 57 ; 67 ; 77) relié électriquement avec la ou l'au moins une piste conductrice, qui est disposé sur la surface libre du support (13 ; 73) ou de l'enveloppe (15 ; 75) et est relié avec la piste conductrice incorporée par le biais d'un contact traversant.

4. Ensemble de conducteurs d'électrode selon l'une des revendications précédentes, lequel présente une multiplicité de pistes conductrices (11 ; 71) s'étendant parallèlement et des découpes sur le support (13 ; 73) et également dans l'enveloppe (15 ; 75) adaptées à l'évolution des pistes conductrices.

5. Ensemble de conducteurs d'électrode selon la revendication 4, lequel présente un agencement (50) comme un filet de pistes conductrices se croisant et présente essentiellement des découpes en polygones dans le support et éventuellement également dans l'enveloppe.

6. Ensemble de conducteurs d'électrode selon la revendication 5, doté d'un réseau en forme de matrice de pôles d'électrodes qui sont disposés en particulier à des points d'intersection ou de liaison de plusieurs pistes conductrices.

7. Ensemble de conducteurs d'électrode selon l'une des revendications précédentes, dans lequel le support (13 ; 73) et/ou l'enveloppe (15 ; 75) présente un polymère à cristaux liquides, LCP, ou un polyimide, PI.

8. Ensemble de conducteurs d'électrode selon l'une des revendications précédentes, dans lequel l'épaisseur du support (13 ; 73) et/ou de l'enveloppe (15 ; 75) se situe dans la plage entre 5 et 100 µm et l'épaisseur de la piste ou de chaque piste conductrice (11 ; 71) se situe dans la plage entre 0,1 et 20 µm.

9. Ensemble de conducteurs d'électrode selon l'une des revendications précédentes, dans lequel la largeur de la piste ou de chaque piste conductrice (11 ; 71) se situe dans la plage entre 2 et 500 µm et la largeur locale du support (13 ; 73) est supérieure à la largeur de la piste conductrice et se situe dans la plage entre 25 et 250 µm.

10. Ensemble de conducteurs d'électrode selon la revendication 1, dans lequel la base (40 ; 50 ; 60) présente un matériau en polyuréthane, en silicone ou en caoutchouc.

11. Ensemble de conducteurs d'électrode selon la revendication 1 ou la revendication 10, dans lequel la base (40 ; 50 ; 60) est de forme cylindrique ou comme un ballon, ou présente un segment cylindrique ou en forme de ballon.

12. Implant médical (50 ; 60 ; 70), notamment ligne d'électrode, tapis d'électrodes ou cathéter à ballonnet doté d'un ensemble de conducteurs d'électrodes (10 ; 20 ; 30 ; 52 ; 62) selon l'une des revendications précédentes.

13. Implant médical (70) selon la revendication 12, dans lequel l'ensemble de conducteurs d'électrode se voit associé à un composant fonctionnel (90) supplémentaire, notamment un composant de commande électronique et/ou d'exploitation et celui-ci est en particulier placé entre l'ensemble de conducteurs d'électrode et une base (80) associée.
